# EUROPÄISCHE PATENTANMELDUNG

(11) **EP 0 658 353 A1**
(43) Veröffentlichungstag der Anmeldung: **21.06.1995**
(21) Anmeldenummer: 93118655.5
(22) Anmeldetag: 19.11.1993
(51) Int. Cl.: A61M 1/34, A61M 1/16, A61M 1/28, A61K 31/19

(54) **Pyruvathaltige CAPD- und Substitutionslösungen sowie pyruvathaltige Dialysierflüssigkeiten**

(30) Priorität: 19.11.1993 DE
(71) Anmelder: Reinhardt, Bertold, Dr., D-61440 Oberursel (DE)
(72) Erfinder: Reinhardt, Bertold, Dr., D-61440 Oberursel (DE)

(57) **Zusammenfassung**

Es werden neue Dialysierlösungen zur Blutreinigung durch alle Arten der Nierenersatztherapie, insbesondere der Peritonealdialyse sowie Substitutionslösungen für die Verwendung bei der Hämfiltration und Hämodiafiltration beschrieben, die als Puffersubstanz Natriumpyruvat enthalten, eine Substanz, die eine ausreichende Korrektur der metabolischen Acidose ermöglicht und in ihrer Anwendung Vorteile auf das Makrophagensystem und auf die Zellen des Peritoneums beinhaltet.

## Beschreibung

Die Erfindung betrifft eine Natriumpyruvat enthaltende Dialysierflüssigkeit zur Blutreinigung durch jede Art von Nierenersatztherapie wie die Hemodialyse, Hemofiltration, CAVH, CAVHD, insbesondere aber der Peritonealdialyse (CAPD) und ihr verwandter Methoden. Die Erfindung betrifft ferner Pyruvat enthaltende Substitutionslösungen wie sie bei der Hemofiltration und der Hemodiafiltration verwendet werden.

Neben der Entfernung von harnpflichtigen Substanzen aus dem Blut, der Bilanzierung der Elektrolyte, ist eine der wichtigsten Aufgaben der Nierenersatztherapie die Korrektur der metabolischen Azidose. Beim Abbau von Eiweißen entsteht als Endprodukt Harnstoff und Säure, die normalerweise über die Nieren ausgeschieden bzw. neutralisiert werden.

Bei niereninsuffizienten Patienten kommt es aber zu einer pathologischen Ansäuerung des Blutes. Um diesen Säureüberschuß zu beseitigen, enthalten die in den Nierenersatztherapien verwendeten Spüllösungen einen Puffer.

Sowohl in der Hämodialyse als auch in der CAPD wurde zunächst Bicarbonat in Form von Natriumbicarbonat verwendet und nach anfänglichen Versuchen durch einen anderen Puffer wie Lactat (meist in der CAPD verwendet) oder Acetat (meist in der Dialyse verwendet) ersetzt.

Der Grund, den natürlichen Puffer Bicarbonat durch Acetat oder Lactat zu ersetzen, lag darin, daß man zunächst technisch nicht in der Lage war, die Stabilitiät der Bicarbonatlösungen zu beherrschen.
Nach Verbesserung der Technologie hat Bicarbonat als Puffer in der Hämodialyse, Hämofiltration und Hämodiafiltration breiten Einsatz gefunden, wobei die Vermeidung der Ausfällung von Calciumcarbonat immer noch nicht vollständig beherrscht wird.

Ähnliches gilt für die Stabilität der Bicarbonat enthaltenden Lösung während der Produktion und während des Transportes.

In neueren Entwicklungen ist der Einsatz von Bicarbonat in CAPD-Lösungen in Vorbereitung bzw. in klinischer Prüfung. Dabei ist jedoch zu bedenken, daß aus therapeutischen Gründen diese Lösungen bis zu 2 mmol/l Calcium und bis zu 42 mmol/l Bicarbonat enthalten können. Unter diesen Bedingungen, aber auch bereits bei weit niedrigeren Bicarbonat- und Calciumkonzentrationen, ist das Löslichkeitsprodukt für Calciumcarbonat überschritten, so daß die Möglichkeit einer Calciumcarbonatausfällung gegeben ist. Das temporäre Ausbleiben der Calciumcarbonatprezipitation solcher Lösungen beruht auf den Eigenschaften von chemischen Gleichgewichten in übersättigten Lösungen. Ob jedoch solche im Labor hergestellten Lösungen ihre Gleichgewichtslage im Peritonealraum des Patienten beibehalten, muß erst noch unter Alltagsbedingungen nachgewiesen werden.

Darüber hinaus haben die in Vorbereitung befindlichen Lösungen einige Handhabungsnachteile gegenüber nicht bicarbonathaltigen Lösungen: sie müssen nämlich im Zweikammer-Beutelsystem getrennt aufbewahrt werden. Dabei wird in einer Beutelkammer die Natrium-, Magnesium-, Calciumchlorid- und Glukosemonohydrat enthaltende Lösung und in der zweiten Beutelkammer die Natriumbicarbonat haltigen Lösung aufbewahrt.

Dies geschieht aus zwei Gründen:
1. Um eine Calciumcarbonatausfällung während der Sterilisation (meist Autoklavierung) zu verhindern,
2. um eine längerfristige Überschreitung des maximalen Ionenproduktes zu vermeiden, was zu einer Ausfällung an Calciumcarbonat führen kann.

Durch die Aufbewahrung im Zweikammer-Beutelsystem muß die Lösung kurz vor der Applikation gemischt werden, nachdem zwei ventilähnliche Sollbruchstellen, die die Lösung A und B trennen, geöffnet wurden. Dies stellt insbesondere für einen Teil der Patienten ein Handhabungsproblem dar.

Der hier angemeldeten Erfindung lag daher die Aufgabe zugrunde, eine Substanz zu finden, die bei der praktischen Anwendung leichter zu handhaben sind, die die physiologischen Nachteile von Lactat vermeidet, die zu einer annehmnaren Korrektur der metabolischen Acidose führt und die in ihrer Verwendung Vorteile auf das Makrophagensystem (Abwehrsystem) und auf die Zellen des Peritoneums (Bauchfell) beinhaltet.

Bei Patienten, die mit konventioneller Peritonealdialyse behandelt werden, tritt, belegt in der entsprechenden Fachliteratur, alle 12 - 36 Monate Peritonitis (Bauchfellentzündung) auf. Die Bauchfellentzündungen entstehen meist durch eine Kombination aus externer Kontamination und einer toxischen Wirkung von konventionellen Dialysierflüssigkeiten auf die peritoneale Zellfunktion: die Peritonealzellen werden durch den Kontakt mit konventionellen Spüllösungen in ihrer Funktion zum einen unspezifisch reduziert, in ihrer Immunantwort (Abwehrverhalten) gegenüber pathogenen Keimen aber spezifisch reduziert.

Der klinisch relevanteste Parameter zur Testung von gestörten Zellfunktionen durch die Toxizität, z. B. von Peritonealdialysierlösungen, sind Vitalitätsprüfungen der Zellen, Zellwachstum, Chemotaxis, Phagozytose und die Cytokinproduktion. Als einer der Hauptparameter für die cytotoxische Wirkung von Peritoneal-spüllösungen auf die Zellen gelten die Puffer-zusätze Lactat und Acetat.

Der Vorteil von pyruvathaltigen Lösungen liegt im physiologischen Bereich darin, die Puffer Lactat, und Acetat durch Pyruvat zu ersetzen, und ebenso zu einer ausreichenden Korrektur der metabolischen Acidose zu gelangen, wie das bei den anderen Puffern der Fall ist.
Pyruvat wird durch die Peritonealzellen resorbiert und in der Leber zu Azetyl-Coenzym-A und den Folgeprodukten des Zitratzyklus abgebaut. Dabei entstehen genügend Kohlen-dioxidäquivalente, die in Bicarbonat umgewandelt werden und eine ausreichende Korrektur der metabolischen Azidose garantieren.
Der Unterschied in der Verwendung von Pyruvat zu Lactat oder Bicarbonat liegt darin, daß durch Pyruvat weder die spezifische noch die unspezifische Zellfunktion beeinträchtigt werden und die Handhabungsnachteile der Bicarbonatlösung vermieden werden.

Pyruvat wird als Natriumsalz der Lösung zugegeben in Konzentrationsbereichen von 1 mmol/l bis 100 mmol/l, insbesondere aber im Bereich von 25 - 40 mmol/l.

Die Substanz Pyruvat kann anstelle von Acetat oder Bicarbonat oder aber in Kombination mit diesen Substanzen auch in Spüllösungen eingesetzt werden, wie sie in der Hemodialayse und der ihr verwandten Verfahren verwendet werden.

In der Hämodialyse und in den mit ihr verwandten Verfahren werden schon seit Jahren Bicarbonat als Puffer enthaltende Dialysierflüssigkeiten hergestellt, und zwar so, daß einerseits ein basisches Bicarbonatkonzentrat und andererseits ein saures, Calcium enthalten-des Elektrolytkonzentrat in getrennten Behältern verwendet werden. Diese Behälter werden an Dialysemaschinen oder neuerdings auch an Zentralversorgungen angeschlossen und erst in der Dialysemaschine, also unmittelbar vor Gebrauch, aus den beiden Konzentraten und Wasser zur endgültigen Dialysierflüssigkeit über Proportionierungssysteme gemischt.

Selbst bei dieser Art der Herstellung kommt es insbesondere in den Dialysemaschinen trotz zusätzlicher technischer Verbesserungen zu Calciumcarbonatausfällungen, was langfristig zu Komplikationen in diesen Maschinen führen kann.

Dies gilt um so mehr, als die Calciumcarbonatprezipitation durch Zugabe von Säuren in regelmäßigen Abständen entfernt werden. Diese Zugabe von Säuren (Essigsäure und andere verdünnte Säuren) greifen das Material in den Dialysemaschinen an.

Diese technischen Einschränkungen bei der Verwendung von Bicarbonat als Puffer werden deshalb in Kauf genommen, weil der ansonsten in der Dialyse ohne technische Komplikationen verwendete Puffer Acetat Nachteile (Nebenwirkungen), u.a. auf die Hämodynamik des Patienten hat.

Die Eigenschaften einer Pyruvat enthaltenden Spüllösung in der Hemodialyse sind dadurch charakterisiert, daß die in den heute verwendeten Lösungen enthaltenen Substanzen Bicarbonat und/oder Acetat durch Pyruvat ersetzt werden, von Pyruvat eine dem Bicarbonat vergleichbare Pufferwirkung erwartet werden kann, eine Calciumcarbonatausfällung wie bei der Verwendung von Bicarbonatlösungen auftreten kann vermieden wird und das nicht nebenwirkungsfreie Acetat ersetzt wird.
Außerdem kann eine Pyruvat enthaltende Spüllösung in nur einem Konzentratkanister bereitgestellt werden.
Außerdem muß eine Pyruvat enthaltende Lösung nicht mit z. B. Kohlendioxideinleitung angesäuert werden, wodurch ein Aufblähen der Kanister, insbesondere bei höheren Temperaturen, vermieden werden kann.

Die Erfindung im Bereich der Substitutionslösungen:
Auf den Bereich der Substitutionslösungen sind die klinisch-physiologischen und technischen Modalitäten der Puffer Lactat, Acetat und Bicarbonat sinngemäß übertragbar. Auch hier werden zur Zeit Bicarbonat, Lactat und teilweise auch Acetat als Puffer verwendet. Auch in diesen Lösungen führt der Einsatz von Pyruvat anstelle von Bicarbonat, Lactat und Acetat zu den bereits beschriebenen Vorteilen, wie Ausbleiben einer Calciumcarbonatausfällung, Anlieferung der Substitutionslösung in nur einem Beutel bei lang anhaltender Stabilität.

Die im folgenden aufgeführten Beispiele sollen die Erfindung für den jeweiligen Bereich erläutern.
Zusammensetzungsbeispiel für eine Peritonealdialyselösung
Es werden 35 mmol/l Natrium-Pyruvat,
2,35 mmol/l NaCl,
1,5 mmol CaCl₂ x 2 H₂O,
0,5 mmol/l MgCl₂ x 6 H₂O
und 0,83 mmol/l Glukosemonohydrat
eingewogen. Anschließend wird der pH-Wert mit 1 HCl auf einen pH-Wert von 5,5 eingestellt und die so hergestellte Lösung pyrogenfrei filtriert und anschließend hitzesterilisiert.

Fertige Dialysierflüssigkeiten für die Hämodialyse können beispielsweise folgende Zusammensetzung haben:

| | |
|---|---|
| Na⁺ | 130 - 145 mval/l |
| Mg⁺⁺ | 0 - 3 mval/l |
| K⁺ | 0 - 4 mval/l |
| Ca⁺⁺ | 0,5 - 4 mval/l |
| Cl⁻ | 90 - 125 mval/l |
| Pyruvat | 25 - 40 mval/l |

Substitutionslösungen können beispielsweise folgende Zusammensetzung haben:

| | |
|---|---|
| Na⁺ | 130 - 150 mmol/l |
| Mg⁺⁺ | 0,5 - 1,5 mmol/l |
| K⁺ | 0 - 4 mmol/l |
| Ca⁺⁺ | 1,0 - 2 mmol/l |
| Cl⁻⁻ | 90 - 125 mmol/l |
| Pyruvat | 25 - 45 mmol/l |

## Patentansprüche

1. Dialysierflüssigkeiten zur Blutreinigung durch alle Arten der Nierenersatztherapieverfahren, insbesondere aber der Peritonealdialyse sowie Substitutionslösungen wie sie bei der Hämofiltration und Hämodiafiltration verwendet werden, dadurch gekennzeichnet, daß sie Natriumpyruvat als Puffersubstanz enthalten.

2. Lösungen nach Anspruch 1, dadurch gekennzeichnet, daß die Pyruvatkonzentration der Lösungen im Bereich von 1 - 100 mmol/l liegt, bevorzugt im Bereich von 20 - 40 mmol/l.

3. Lösungen nach Anspruch 1 - 3, dadurch gekennzeichnet, daß Pyruvat vergleichbare Pufferwirkung wie Lactat und Acetat hat, ohne aber eine toxische Wirkung auf die Peritonealzellen und das Immunsystem zu haben.

4. Lösungen nach Anspruch 1 - 2, dadurch gekennzeichnet, daß Pyruvat mit anderen Puffern wie Lactat, Acetat und Bicarbonat in Konzentrationen, wie unter Anspruch 3 aufgeführt, kombiniert werden.

5. Lösungen nach Anspruch 1 - 2, dadurch gekennzeichnet, daß der pH-Wert dieser Lösungen bei Applikation alle pH-Bereiche haben kann, die physiologisch verträglich sind, insbesondere den pH-Bereich von pH 5,0 bis pH 8.

6. Die pyruvathaltigen Lösungen können sowohl in Einkammersystemen als auch in Mehrkammersystemen verwendet werden
